# EUROPÄISCHE PATENTSCHRIFT

(11) **EP 1 524 954 B1**
(45) Veröffentlichungstag und Bekanntmachung des Hinweises auf die Patenterteilung: **27.02.2019**
(21) Anmeldenummer: 03766292.1
(22) Anmeldetag: 25.07.2003
(51) Int. Cl.: A61F 2/38

(54) **KNIEPROTHESE**
KNEE PROSTHESIS
PROTHESE DU GENOU

(30) Priorität: 26.07.2002 EP 02016768
(43) Veröffentlichungstag der Anmeldung: 27.04.2005
(73) Patentinhaber: WALDEMAR LINK GmbH & Co. KG, 22339 Hamburg (DE)
(72) Erfinder: KELLER, Arnold, 23863 Kayhude (DE)
(74) Vertreter: Glawe, Delfs, Moll
(86) Internationale Anmeldenummer: PCT/EP2003/008196
(87) Internationale Veröffentlichungsnummer: WO 2004/012633

(56) Entgegenhaltungen:
- EP-A- 0 420 460
- DE-A- 2 901 009
- DE-A- 4 102 509

## Beschreibung

Für den Ersatz des menschlichen Kniegelenks verwendet man Prothesentypen, deren femorale und tibiale Teile je nach dem Erhaltungszustand des Bandapparats eine geringere oder stärker ausgeprägte gegenseitige Zwangsführung aufweisen. Diese bezieht sich auf die wesentlichen Bewegungsfreiheitsgrade des Knies, nämlich die Beugebewegung um eine Querachse, die Rotationsbewegung um eine etwa parallel zur Schienbeinrichtung verlaufende Rotationsachse und eine translatorische Bewegung in AP-Richtung (AP = anteroposterior). Den geringsten Grad an gegenseitiger Zwangsführung weisen sogenannte ungekoppelte Prothesen auf, die lediglich aus einem femoralen Kondylenpaar und einer tibialen Gleitfläche bestehen. Sie werden bei gut erhaltenem Bandapparat verwendet. Das andere Extrem bilden Scharnierprothesen, die bei schlecht erhaltenem Bandapparat verwendet werden und die Bewegungsmöglichkeiten des Knies auf die Beugebewegung einschränken (EP-A-42 04 60, DE-OS-29 01 009).Zwischen diesen Extremen gibt es in unterschiedlichem Maße teilgekoppelte Systeme, die zwischen dem femoralen und dem tibialen Teil einen Zwischenteil aufweisen, der durch Bildung eines Rotationslagers die Führungsaufgaben für die Rotationsbewegung übernimmt.

Unter den teilgekoppelten Prothesen, die eine Rotationsbewegung erlauben, sind zwei Typen zu unterscheiden. Bei dem ersten Typ wird die gesamte Last über das Zwischengelenkstück übertragen, das im Verhältnis zum tibialen Teil ein Rotationslager und gegenüber dem femoralen Teil ein Beuge-Scharniergelenk bildet (DE-C-26 60 623). Da in diesem Fall die Kondylengleitflächen lediglich für eine Beugebewegung bestimmt sind, können sie kongruent zu den Gegenflächen ausgebildet sein. Die Gegenflächen sind daher konkav mit gleichem Krümmungsradius gestaltet. Der zweite Typ teilgekoppelter Prothesen überträgt die Last nicht über das Gelenkzwischenstück, sondern unmittelbar von den Kondylengleitflächen auf damit zusammenwirkende tibiale Gleitflächen (EP-A-174 531). In diesem Fall findet zwischen den Kondylengleitflächen und den Tibiagleitflächen nicht nur eine Beugebewegung, sondern auch die Rotationsbewegung statt. Daher dürfen die Tibiagleitflächen nicht kongruent zu den Kondylengleitflächen ausgebildet sein. Wenn sie eine freie Rotationsbewegung ermöglichen sollen, müssen die Tibiagleitflächen eben sein. In der Regel läßt man sie jedoch vor demjenigen Bereich, in welchem sie mit den Kondylenflächen bei gleicher AP-Ausrichtung des Femurteils und des Tibiateils zusammenwirken (Bereich normalen Kontakts), nach vorne hin ein wenig ansteigen. Dies hat zur Wirkung, daß bei einer Rotation diejenige Kondylengleitfläche, die sich gegenüber der Tibiagleitfläche bei der Rotation nach vorne verschiebt, angehoben wird. Dies erzeugt unter der vom Gelenk übertragenen Last ein rückdrehendes Moment, das dafür sorgt, daß die Prothesenteile, sobald dies möglich ist, wieder in ihre Normalstellung gleicher AP-Ausrichtung zurückkehren. Bei der Rotation gegenüber dem Tibiateil und der dadurch bewirkten Anhebung des Femurteils verliert die nach hinten wandernde Kondylengleitfläche ihren Kontakt mit der Tibiagleitfläche verliert. Die gesamte Last muß dann auf der anderen Seite übertragen werden, was nicht zu erhöhtem Verschleiß, sondern auch zu einem unerwünschten Biegemoment im Bereich des Rotationslagers führt. Von diesem Stand der Technik, der im Oberbegriff des Anspruchs 1 genannt ist, geht die Erfindung aus.

In einer bekannten Veröffentlichung (DE-A-41 02 509) ist eine teilgekoppelte Prothese erläutert, bei welcher sowohl die Beugebewegung als auch die Rotationsbewegung sich zwischen den femoralen und tibialen Gleitflächen abspielt. Die femoralen Gleitflächen sind in der Sagittal- und Frontalebene konvex gerundet. Die tibialen Gleitflächen sind Mulden, die mit der Form der femoralen Gleitflächen komplementär identisch sind. Im Falle einer Rotation heben sich die femoralen Gleitflächen aus den tibialen Mulden heraus und liegen nur noch auf den vorderen bzw. hinteren Kanten dieser Mulden auf und nicht mehr an den Innenflächen der Mulden. Dies führt zu hoher punktueller Belastung und Verformung sowie Verschleiß. Der Widerstand gegen Rotation ist bei beginnender Rotation sehr hoch und fällt bei fortschreitender Rotation ab. Dies ist nachteilig, weil es den natürlichen Verhältnissen widerspricht. Erwünscht ist stattdessen ein gleichmäßiger Anstieg der Kräfte, die der Rotation entgegenwirken und das Gelenk nach Beendigung der Rotation sanft in seine Normalstellung zurückführen. Einen Hinweis darauf, wie die Gleitflächen gestaltet werden müssen, damit sie sowohl Rotationsmöglichkeit als auch eine sanfte Stabilisierung der Rotation gewähren und daß im Falle von Rotation eine die Prothese schonende Kraftübertragung stattfinden kann, läßt sich der Veröffentlichung nicht entnehmen.

Ausgehend von dem im Oberbegriff des Anspruchs 1 genannten Stand der Technik liegt der Erfindung die Aufgabe zugrunde, die Kraftübertragung zwischen den Prothesenkomponenten im Falle von Rotation um die Tibialängsachse zu verbessern. Die Lösung liegt in dem Merkmal des Anspruchs 1.

Demnach steigen die Tibiagleitflächen auch hinter den Bereichen des normalen Kontakts an, und zwar dergestalt, daß im Falle von Rotation jede der beiden Kondylengleitflächen mit der zugehörigen Tibiagleitfläche in Berührung bleibt; die eine Kondylengleitfläche steht in Kontakt mit dem ansteigenden Teil der Tibiagleitfläche vor dem Bereich normalen Kontakts, die andere mit dem nach hinten ansteigenden Bereich.

Besonders einfach und übersichtlich sind die geometrischen Verhältnisse dann, wenn der Krümmungsradius des mit der Tibiagleitfläche zusammenwirkenden Teils der Kondylengleitflächen in der Beugeebene im wesentlichen konstant ist, d.h. wenn die Kondylengleitflächen kreisbogenförmig gestaltet
sind. Die Erfindung ist aber auch anwendbar, wenn dies nicht der Fall ist. Wenn der Verlauf der Kondylenflächen unregelmäßig ist, ist es dabei allerdings zweckmäßig, eine relative Bewegungsmöglichkeit der femoralen und tibialen Teile in AP-Richtung vorzusehen. Dies ist nicht notwendig, wenn die Kondylengleitflächen nach einer archimedischen Spirale geformt sind. Das Profil der Kondylengleitflächen sollte im allgemeinen konstant sein

Die Erfindung wird im folgenden näher unter Bezugnahme auf die Zeichnung erläutert, die ein vorteilhaftes Ausführungsbeispiel veranschaulicht. Es zeigen:
- Fig. 1: eine Seitenansicht der Prothese,
- Fig. 2: eine Draufsicht auf die tibialen Gleitflächen,
- Fig. 3: einen Schnitt durch die tibialen Gleitflächen und
- Fig. 4 und 5: zwei Seitenansichten von entgegengesetzten Seiten bei rotiertem Femurteil.

Die Prothese weist einen Femurteil 1 und einen Tibiateil 2 auf, die über Dornen 3, 4 in bekannter Weise am unteren Ende des Oberschenkelknochens (Femur) bzw. am oberen Ende des Schienbeins (Tibia) zu verankern sind. Der Femurteil 1 weist ein Paar von Kondylengleitflächen 5 auf, die sich an der Vorderseite zu einer Patellagleitfläche 6 vereinigen. Der Tibiateil 2 bildet oben eine Tragplatte 7, auf der das sogenannte Tibiaplateau 8 aus einem gleitgünstigen Werkstoff, beispielsweise Polyethylen, verankert ist, das die Tibiagleitflächen 9 bildet, auf denen die vorzugsweise von poliertem Metall gebildeten Kondylengleitflächen 5 gleiten. Der Femurteil 1 und der Tibiateil 2 sind miteinander durch einen Zwischenteil 10 gekoppelt, der einerseits mit dem Femurteil 1 ein Beugelager mit der Achse 11 und andererseits mit dem Tibiateil ein Rotationslager mit der Achse 12 bildet. Einzelheiten dieser Konstruktion sind erläutert in den europäischen Patentanmeldungen 1 110 261 und 1 111 551, auf die hiermit Bezug genommen wird und deren Offenbarungsinhalt zum Gegenstand der vorliegenden Anmeldung gemacht wird.

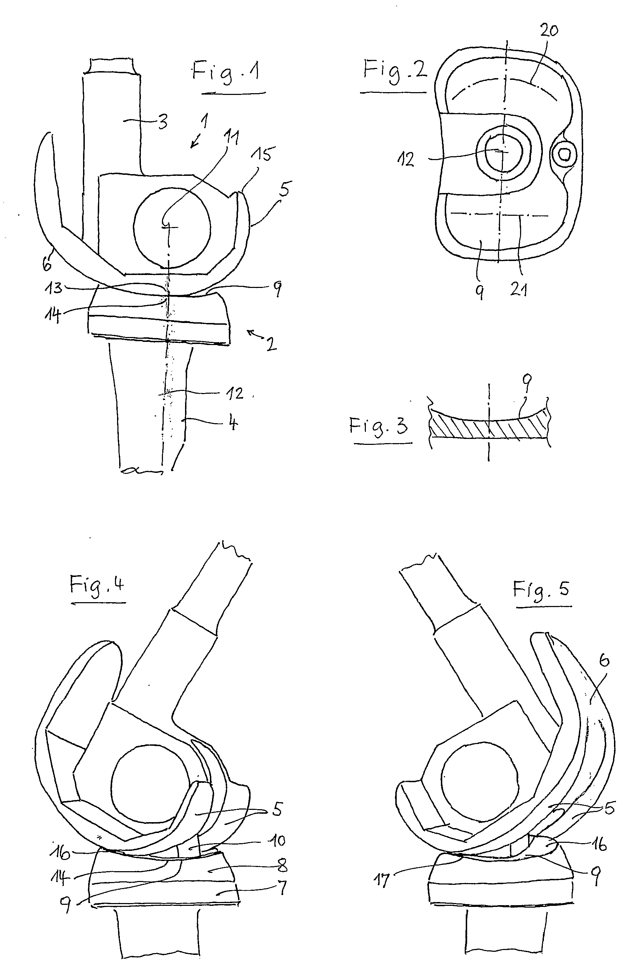

In unrotierter Stellung (Fig. 1) ruhen die femoralen Gleitflächen 5 mit ihrem Bereich 13, dessen Richtung etwa lotrecht zum Radius verläuft, auf dem Bereich 14 normalen Kontakt der tibialen Gleitflächen 9, dessen Richtung etwa lotrecht zur Achse 12 verläuft. Bei Beugebewegung kann der zwischen dem Bereich 13 und dem hinteren Ende 15 der femoralen Gleitflächen liegende Abschnitt tragend mit der Tibiagleitfläche 9 in Berührung kommen. Dieser Abschnitt verläuft im dargestellten Beispiel kreisbogenförmig mit konstantem Radius zur Beugeachse 11. Das Gleitflächenprofil ist in diesem Abschnitt konstant.

Der Bereich 14 der Tibiagleitfläche 9 hat dasselbe Profil (im Frontalschnitt) wie der damit zusammenwirkende Abschnitt 13-15 der zugehörigen Femurgleitfläche. Das bedeutet, daß im unrotierten Zustand theoretischer Linienkontakt herrscht. Praktisch ergibt sich Flächenkontakt infolge der Nachgiebigkeit des Werkstoffs der Tibiagleitflächen 9.

Der vor dem Bereich 13 liegende Abschnitt 6 der Kondylengleitflächen und der Patellagleitfläche ist für die Übertragung der Lastkräfte auf den Tibiateil 2 der Prothese ohne Belang.

Die Tibiagleitfläche 9 ist in der Sagittalebene schwach konkav gekrümmt, wie es Fig. 3 zeigt. Der Krümmungsradius ist beträchtlich größer als der Radius des femoralen Gleitflächenabschnitts 13-15. Dies ist erforderlich, damit die femoralen Gleitflächen im Falle von Rotation sich - ausgehend von dem Bereich normalen Kontakts 14 - im wesentlichen ungehindert um eine geringe Distanz vor- und zurückbewegen können. Im Falle einer kräftigen Rotation verlassen die Kondylengleitflächen 5 den Bereich 14 normalen Kontakts. Auf der einen Seite (siehe Fig. 4) bewegen sie sich in den ansteigenden Abschnitt 16 der Tibiagleitflächen, der vor dem Bereich normalen Kontakts 14 liegt. Auf der anderen Seite (Fig. 5) bewegen sie sich im hinteren, ansteigenden Abschnitt 17 der Tibiagleitflächen 9.

Die Tibiagleitflächen sind so geformt, daß die Kondylengleitflächen 5 im Falle einer solchen Rotation auf beiden Seiten Kontakt mit der Tibiagleitfläche 9 behalten, nämlich auf der einen Seite mit dem vorderen Abschnitt 16 und auf der anderen Seite mit dem hinteren Abschnitt 17.

Will man in diesen Abschnitten Linienkontakt zwischen den Kondylengleitflächen 5 und den Tibiagleitflächen 9 aufrecht erhalten, so muß man die Tibiagleitflächen 9 so formen, daß sie in Richtung von Kreisbögen 20 um die Rotationsachse 12 in einer Schnittebene, die diese Rotationsachse enthält, dasselbe Profil wie die Kondylengleitflächen 5 aufweisen. Dies läßt sich ohne weiteres mit Hilfe eines Werkzeugs bewerkstelligen, das das Profil der Kondylengleitflächen aufweist und um die Achse 12 rotiert wird. Dies ist jedoch verhältnismäßig aufwendig. Einfacher ist es, die Tibiagleitflächen 9 mittels Werkzeugen zu fräsen, die in AP-Richtung 20 bewegt werden. In diesem Fall verzichtet man bei Rotation auf den idealen Linienkontakt zwischen den Kondylengleitflächen 5 und den Tibiagleitflächen 9 um so stärker, je weiter sich der Punkt des jeweiligen Kontakts von dem Bereich 14 normalen Kontakts entfernt. Dies ist aber unschädlich, weil so starke Rotation vergleichsweise selten stattfindet und die Perioden lang dauernder Lastübertragung auf den Bereich 14 normalen Kontakts beschränkt sind. Entscheidend ist, daß bei einer solchen starken Rotation nicht nur eine der beiden Kondylengleitflächen mit der Tibiagleitfläche zusammenwirkt, sondern beide.

## Patentansprüche

1. Knieprothese mit einem femoralen Prothesenteil (1), der ein Paar von Kondylengleitflächen (5) bildet, einem tibialen Teil (2), der mit den Kondylengleitflächen (5) zusammenwirkende Tibiagleitflächen (9) aufweist, sowie einem Kupplungsteil (10), der die femoralen und tibialen Teile (1, 2) durch Bildung eines Beugegelenks mit dem,femoralen Teil (1) und eines Rotationsgelenks mit dem tibialen Teil (2) miteinander verbindet, wobei das Rotationsgelenk eine zum Schienbein etwa parallele Rotationsachse (12) hat, die *in AP-Richtung* gegenüber *den femoralen und tibialen* Prothesenteilen (1, 2) fest angeordnet ist, und wobei die Tibiagleitflächen einen Bereich (14) normalen Kontakts aufweisen, welcher mit der zugehörigen Kondylengleitfläche (5) bei gleicher AP-Ausrichtung der femoralen und tibialen Teile (1, 2) zusammenwirkt und vor dem Bereich (14) normalen Kontakts mit einem Krümmungsradius ansteigen, **dadurch gekennzeichnet, daß** dieser Krümmungsradius wesentlich größer als der Krümmungsradius des mit der Tibiagleitfläche zusammenwirkenden Teils (13-15) der Kondylengleitfläche (5) ist, und die Tibiagleitflächen (9) auch hinter dem Bereich (14) normalen Kontakts mit einem Krümmungsradius ansteigen der wesentlich größer ist als der Krümmungsradius des mit der Tibiagleitfläche zusammenwirkenden Teils (13-15) der Kondylengleitfläche, und zwar dergestalt, daß im Falle von Rotation jede der beiden Kondylengleitflächen (5) mit der zugehörigen Tibialgleitfläche (9) vor bzw. hinter dem Bereich (14) normalen Kontakts in Berührung bleibt.

2. Prothese nach Anspruch 1, **dadurch gekennzeichnet, daß** der Krümmungsradius des mit der Tibiagleitfläche (9) zusammenwirkenden Teils (13-15) der Kondylengleitfläche (5) in der Beugeebene im wesentlichen konstant ist.

## Claims

1. Knee prosthesis with a femoral prosthetic part (1) which forms a pair of condylar sliding surfaces (5), with a tibial part (2) which has tibial sliding surfaces (9) cooperating with the condylar sliding surfaces (5), and also a coupling part (10) which connects the femoral and tibial parts (1, 2) to one another by forming a flexion joint with the femoral part (1) and a rotation joint with the tibial part (2), the rotation joint having a rotation axis (12) approximately parallel to the tibia, which rotation axis (12) is fixed in relation to the femoral and tibial prosthesis parts (1, 2) in the anteroposterior direction, and the tibial sliding surfaces having an area (14) of normal contact which, when the femoral and tibial parts (1, 2) have the same anteroposterior alignment, cooperates with the associated condylar sliding surface (5), and, in front of the area (14) of normal contact, they slope upward with a radius of curvature, **characterized in that** this radius of curvature is much greater than the radius of curvature of that part (13-15) of the condylar sliding surface (5) cooperating with the tibial sliding surface, and the tibial sliding surfaces (9) also slope upward behind the area (14) of normal contact with a radius of curvature which is much greater than the radius of curvature of that part (13-15) of the condylar sliding surface cooperating with the tibial sliding surface, specifically in such a way that, in the event of rotation, each of the two condylar sliding surfaces (5) remains touching the associated tibial sliding surface (9) in front of or behind the area (14) of normal contact.

2. Prosthesis according to Claim 1, **characterized in that** the radius of curvature of that part (13-15) of the condylar sliding surface (5) cooperating with the tibial sliding surface (9) is substantially constant in the flexion plane.

## Revendications

1. Prothèse du genou avec une partie de prothèse fémorale (1), qui forme une paire de surfaces de glissement condyliennes (5), une partie tibiale (2), qui présente deux surfaces de glissement tibiales (9) coopérant avec les surfaces de glissement condyliennes (5), ainsi qu'avec une partie de couplage (10), qui relie l'une à l'autre les parties fémorale et tibiale (1, 2) par formation d'une articulation flexible avec la partie fémorale (1) et d'une articulation rotative avec la partie tibiale (2), dans laquelle l'articulation rotative présente un axe de rotation (12) sensiblement parallèle au tibia, qui est disposé de façon fixe en direction AP par rapport aux parties de prothèse fémorale et tibiale (1, 2), et dans laquelle les surfaces de glissement tibiales présentent une zone (14) de contact normal, qui coopère avec la surface de glissement condylienne correspondante (5) pour la même orientation AP des parties fémorale et tibiale (1, 2) et monte avec un rayon de courbure devant la zone (14) de contact normal, **caractérisée en ce que** ce rayon de courbure est sensiblement plus grand que le rayon de courbure de la partie (13-15) de la surface de glissement condylienne (5) coopérant avec la surface de glissement tibiale, et les surfaces de glissement tibiales (9) montent également derrière la zone (14) de contact normal avec un rayon de courbure qui est sensiblement plus grand que le rayon de courbure de la partie (13-15) de la surface de glissement condylienne coopérant avec la surface de glissement tibiale, notamment de telle manière qu'en cas de rotation chacune des deux surfaces de glissement condyliennes (5) reste en contact avec la surface de glissement tibiale correspondante (9) devant ou derrière la zone (14) de contact normal.

2. Prothèse du genou selon la revendication 1, **caractérisée en ce que** le rayon de courbure de la partie (13-15) de la surface de glissement condylienne (5) coopérant avec la surface de glissement tibiale (9) est essentiellement constant dans le plan de flexion.
